(19) Europäisches Patentamt / European Patent Office / Office européen des brevets

(11) **EP 3 805 362 A1**

(12) **DEMANDE DE BREVET EUROPEEN**

(43) Date de publication:
**14.04.2021 Bulletin 2021/15**

(51) Int Cl.:
*C12M 3/00* (2006.01)   *C12M 3/06* (2006.01)
*C12M 1/12* (2006.01)   *C12M 1/00* (2006.01)
*C12M 1/42* (2006.01)   *C12N 5/00* (2006.01)

(21) Numéro de dépôt: **20200325.7**

(22) Date de dépôt: **06.10.2020**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **07.10.2019 FR 1911072**

(71) Demandeur: **Commissariat à l'énergie atomique et aux énergies alternatives**
**75015 Paris (FR)**

(72) Inventeurs:
• **RIVERA, Florence**
**38054 GRENOBLE CEDEX 09 (FR)**

• **BOTTAUSCI, Frédéric**
**38054 GRENOBLE CEDEX 09 (FR)**
• **KRAMMER, Thibault**
**38054 GRENOBLE CEDEX 09 (FR)**
• **CARRIERE, Marie**
**38054 GRENOBLE CEDEX 09 (FR)**
• **FEIGE, Jean-Jacques**
**38054 GRENOBLE CEDEX 09 (FR)**
• **BAILLY, Sabine**
**38054 GRENOBLE CEDEX 09 (FR)**

(74) Mandataire: **Brevalex**
**95, rue d'Amsterdam**
**75378 Paris Cedex 8 (FR)**

(54) **MÉTHODE DE FABRICATION D'UNE STRUCTURE VASCULAIRE**

(57) Méthode de fabrication d'une structure vasculaire comportant :

a) la fabrication d'éléments comprenant au moins un noyau comprenant au moins un biopolymère, et des cellules endothéliales et/ou des cellules souches progénétrices endothéliales sur sa périphérie externe,

b) mise en place desdits éléments dans une chambre fluidique de sorte à ce qu'ils forment au moins un empilement,

c) l'établissement d'un flux de fluide assurant un apport de nutriments et une oxygénation à travers l'empilement de sorte à ménager entre les éléments des canaux le long des lignes de flux.

FIG.3

EP 3 805 362 A1

**Description**

**DOMAINE TECHNIQUE ET ÉTAT DE LA TECHNIQUE ANTÉRIEURE**

La présente invention se rapporte à une méthode de fabrication d'une structure vasculaire, en vue par exemple de fabriquer un tissu vascularisé, voire un organe vascularisé, par exemple pour réaliser une implantation in vivo de cet organe.

Dans le domaine médical, on cherche à réaliser des organes sur puce pour pouvoir étudier leur réaction à des médicaments par exemple. Pour cela les équipes médicales essaient de fabriquer un réseau vasculaire dont le comportement se rapproche au mieux de celui des organes in vivo.

Le réseau vasculaire humain est composé de vaisseaux sanguins comprenant les artères, les artérioles, les veines, les veinules et les capillaires. Il part du cœur et se distribue dans le corps entier. Le réseau vasculaire est chargé de transporter le sang qui permet de libérer l'oxygène, les nutriments, les hormones, les cellules sanguines, les cellules immunitaires, les fluides et la chaleur dans les tissus tout en collectant les déchets. In vivo, les tissus sont perfusés, alimentés par tout un réseau vasculaire et plus précisément des capillaires au niveau des tissus.

Le système vasculaire est complexe et ramifié. Le diamètre intérieur des vaisseaux sanguins peut aller de quelques microns de diamètre, de l'ordre de 5 $\mu$m, à plusieurs millimètres pour les artères, de l'ordre de 25 mm. Les caractéristiques de ces vaisseaux sont variées et leur composition également, celles-ci dépendant principalement de leur fonction finale.

La fabrication d'un réseau vasculaire artificiel est donc très complexe.

Il existe différentes techniques pour tenter de fabriquer une structure se rapprochant d'une structure vasculaire d'un corps humain.

Une technique consiste à recouvrir la paroi d'un canal réalisé dans un substrat en matériau plastique ou en verre, d'hydrogel et de cellules endothéliales. Un flux de liquide traverse le canal et assure l'apport en nutriments et l'oxygénation, permettant aux cellules de se développer et de former des tubes.

Une autre technique consiste à disposer les cellules endothéliales dans un milieu favorable à leur développement, qui vont alors s'auto-arranger jusqu'à former des structures tubulaires en trois dimensions.

Cependant, les structures obtenues par la première technique sont complexes à mettre en œuvre et ne permettent pas d'atteindre des tubes creux endothélialisés de diamètre inférieurs à environ 100 $\mu$m. La seconde technique basée sur l'auto-assemblage des cellules endothéliales permet d'obtenir un réseau de capillaires plus physiologique. Cependant, la formation de ce réseau nécessite plus d'une semaine. Les autres types cellulaires potentiellement inclus dans la construction ne se retrouvent pas alimentés durant cette période et nécrosent, empêchant la construction de tissus épais. C'est pourquoi les dispositifs utilisant cette technique ne dépassent pas la centaine de $\mu$m d'épaisseur.

Il existe également une technique de bioimpression qui consiste à imprimer la structure vasculaire au moyen d'une encre contenant des cellules endothéliales. La méthode de bio-impression par extrusion permet de former des vaisseaux de plusieurs centaines de $\mu$m, par exemple entre 600 $\mu$m et 700 $\mu$m. Or cette méthode est longue et nécessite l'impression d'échafaudages pour maintenir la structure tridimensionnelle. En outre elle applique un stress important aux cellules. En effet, lors de la préparation de l'encre et de l'impression, les cellules endothéliales ne sont pas oxygénées. En outre les limites de résolution de la technique de bioimpression ne permettent pas d'atteindre la taille des capillaires.

Ces techniques ne permettent pas de fabriquer des tissus épais vascularisés. Des modèles de tissus non-vascularisés ont été conçus et implantés in vivo (peau, cartilage). Une artère pulmonaire développée par ingénierie tissulaire a également été transplantée sur une enfant de 4 ans possédant une atrésie pulmonaire. Cela est décrit dans le document *Shin'Oka, 2001, Transplantation of a tissue-engineered pulmonary artery*. Un réseau de tubes creux a également été bioimprimé et implanté chez la souris et a montré une anastomose avec son système vasculaire (*Sooppan, 2015, In vivo anastomosis and perfusion of a 3D printed construct containing microchannel networks*).

**EXPOSÉ DE L'INVENTION**

C'est par conséquent un but de la présente invention d'offrir une méthode de fabrication d'une structure vasculaire complexe, notamment pouvant comporter des capillaires en vue de la réalisation de tissus épais ou d'organes sur puce, ou en vue d'une implantation in vivo de tissus ou d'organes.

Le but mentionné ci-dessus est atteint par une méthode de fabrication comprenant la réalisation d'éléments comprenant un noyau en biopolymère et au moins une couche externe comprenant des cellules endothéliales et/ou des cellules souches progénitrices endothéliales, l'empilement des éléments dans une chambre fluidique comprenant un orifice d'entrée et un orifice de sortie et l'application d'un flux de fluide à travers l'empilement d'éléments, le fluide comprenant au moins des nutriments et assurant l'oxygénation.

La méthode selon l'invention permet de réaliser des tissus épais intégrant des capillaires, par exemple des tissus de plusieurs centaines de $\mu$m d'épaisseur, alors que les méthodes de l'état de la technique ne permettent pas à la fois de réaliser des tissus munis de capillaires et présentant une épaisseur suffisante.

De manière avantageuse, le flux est tel qu'il applique des forces de cisaillements aux cellules favorisant l'attachement des cellules les unes aux autres, et leur élongation le long des lignes de courant.

En choisissant la taille des éléments, il est possible de fabriquer un réseau capillaire ou des artérioles et ou/des veinules, en adaptant l'espacement inter élément qui va définir la taille maximale des vaisseaux sanguins.

De manière très avantageuse, en utilisant plusieurs tailles d'éléments il est possible de fabriquer une structure comprenant des artérioles et des veinules reliées par des capillaires.

Avantageusement le noyau en biopolymère comporte des cellules d'un organe à fabriquer. Ainsi on fabrique directement une structure vascularisée d'un organe donné.

Le biopolymère peut être conservé dans la structure ou être éliminé.

La méthode selon l'invention offre une grande liberté dans le type de structure qui peut être fabriqué en choisissant le ou les types de biopolymères, le ou les types de cellules et les caractéristiques du flux.

En d'autres termes, on réalise un empilement de capsules en biopolymère, pouvant contenir un ou plusieurs types de cellules, les capsules étant recouvertes au moins en partie de cellules endothéliales et/ou des cellules souches progénitrices endothéliales, et on assure un apport de nutriments et une oxygénation par une circulation de fluide à travers l'empilement. Les canaux se forment alors le long des lignes de flux.

La présente invention a alors pour objet une méthode de fabrication d'une structure vasculaire comportant :

a) la fabrication d'éléments comprenant au moins un noyau comprenant au moins un biopolymère, et des cellules endothéliales et/ou des cellules souches progénétrices endothéliales sur sa périphérie externe,
b) mise en place desdits éléments dans une chambre fluidique de sorte à ce qu'ils forment au moins un empilement,
c) l'établissement d'un flux de fluide assurant un apport de nutriments et une oxygénation à travers l'empilement de sorte à ménager des canaux entre les éléments le long des lignes de flux.

Par exemple, le flux de fluide présente un débit tel que les éléments subissent une contrainte de cisaillement moyenne comprise entre 0,1 dyne/cm$^2$ et 100 dynes/cm$^2$, préférentiellement entre 1 dyne/cm$^2$ et 10 dynes/cm$^2$.

Dans un exemple, lors de l'étape a) des cellules sont introduites dans le noyau. Le noyau peut comporter au moins un cœur contenant des cellules et une couche externe contenant des cellules, les cellules du cœur et de la couche externe étant du même type ou non. La couche externe peut contenir des péricytes.

Par exemple, lors de l'étape a), le noyau est réalisé avec un mélange de biopolymères dont l'un au moins offre une bonne affinité avec les cellules endothéliales et/ou des cellules souches progénétrices endothéliales

De préférence, les éléments ont une plus grande dimension inférieure à 1 mm, avantageusement inférieure à 500 $\mu$m.

Les éléments sont avantageusement disposés dans un milieu de culture avant l'étape b) pour permettre la prolifération des cellules endothéliales et/ou des cellules souches progénétrices endothéliales et le recouvrement total ou partiel des noyaux.

Lors de l'étape b), il peut être prévu de mettre en place des éléments des dimensions et/ou de forme différentes. Des éléments de tailles différentes peuvent être répartis en plusieurs empilements successifs dans la direction du flux.

Par exemple, un empilement d'éléments de plus faible taille présentant un espacement inter-éléments de l'ordre du diamètre des capillaires est disposé entre deux empilements d'éléments de plus grande taille présentant un espacement inter-élément de l'ordre du diamètre des artérioles ou veinules.

De manière avantageuse, préalablement à la mise en places des d'éléments dans la chambre fluidique, des cellules endothéliales et/ou des cellules souches progénétrices endothéliales recouvrent les parois de la chambre et/ou des canaux d'entrée et/ou sortie de fluide.

La présente invention a également pour objet un système de fabrication d'une structure vasculaire pour la mise en œuvre de la méthode de fabrication selon l'invention comportant n chambres fluidiques, n ≥ 1, munies d'un orifice d'alimentation en fluide et d'un orifice d'évacuation en fluide, une cavité disposée entre l'orifice d'alimentation et l'orifice d'évacuation et configurée pour recevoir les éléments, et des moyens pour maintenir les éléments dans la cavité, une réservoir de fluide connecté au moins à l'entrée d'alimentation et des moyens de circulation pour assurer la circulation du fluide à travers la cavité.

Le réservoir est avantageusement connecté à l'orifice d'évacuation de sorte à former un circuit fermé.

Selon une caractéristique additionnelle, le système peut comporter des moyens pour isoler la chambre fluidique avant l'arrêt des moyens de circulation.

Selon une autre caractéristique additionnelle, le système de fabrication comporte des moyens pour oxygéner le fluide.

Chaque chambre fluidique peut comporter une partie amovible pour permettre d'accéder à la cavité et retirer la structure vasculaire.

Dans un exemple avantageux, la cavité comporte une partie centrale et des extrémités amont et aval en considérant le sens d'écoulement du fluide, les extrémités amont et aval présentant une section transversale se réduisant en direction des orifices d'alimentation et d'évacuation.

Dans un exemple, n est supérieur ou égal à 2, et le système de fabrication comporte un premier canal connectant en parallèle les chambres fluidiques et un deuxième canal connectant en parallèle les chambres fluidiques, le premier canal et le deuxième canal ayant des diamètres de l'ordre de ceux des artères et des veines.

**BREVE DESCRIPTION DES DESSINS**

La présente invention sera mieux comprise sur la base de la description qui va suivre et des dessins en annexe sur lesquels:

La figure 1A est une représentation schématique d'un exemple d'élément pouvant être mis en œuvre dans le procédé de fabrication.
La figure 1B est une représentation schématique d'un autre exemple d'élément pouvant être mis en œuvre dans le procédé de fabrication.
La figure 1C est une représentation schématique d'un autre exemple d'élément pouvant être mis en œuvre dans le procédé de fabrication.
La figure 1D est une représentation schématique d'un autre exemple d'élément pouvant être mis en œuvre dans le procédé de fabrication.
La figure 2 est une représentation schématique de la réalisation des noyaux en biopolymère,
La figure 3 est une représentation schématique d'une chambre fluidique pour la mise en œuvre de la méthode de fabrication.
La figure 4 est une représentation schématique d'un circuit fluidique adapté pour la mise en œuvre de la méthode de fabrication.
La figure 5 est une vue de la chambre de la figure 3 comportant des éléments.
La figure 6 est une représentation schématique d'un exemple d'empilements d'éléments permettant la fabrication de capillaires, de veinules et d'artérioles.
La figure 7 est une vue similaire à celle de la figure 5 sur laquelle est représenté le flux de fluide.
La figure 8 est une représentation schématique des cellules endothéliales auto-organisées en canaux.
La figure 9 est une photo d'une tranche d'une structure vasculaire obtenue par la méthode de fabrication.
La figure 10 est une vue agrandie de la figure 9.
La figure 11 est une représentation schématique d'un exemple de système de fabrication d'un corps sur puce.

**EXPOSÉ DÉTAILLÉ DE MODES DE RÉALISATION PARTICULIERS**

Dans la présente invention, on entend par « structure vasculaire », une structure comportant un canal ou plusieurs canaux, les dimensions de chaque canal étant du même ordre de grandeur qu'une artère, une veine, une artériole, une veinule ou un capillaire.

On entend par « vaisseau » un canal dans lequel le sang est destiné à circuler, un vaisseau étant une artère, une veine, une artériole, une veinule ou un capillaire. Le canal comporte des parois recouvertes d'un endothélium poreux permettant les échanges avec le milieu environnant. Dans le cas d'un capillaire l'endothélium forme la paroi, dans le cas d'artère et de veine, l'endothélium forme une couche interne du canal recouvrant un ou plusieurs couches externes. La ou les couches externes sont également poreuses permettant les échanges avec le milieu environnant

On entend par « réseau vasculaire », un réseau comprenant des artérioles et des veinules reliées par des capillaires, les artérioles pouvant être reliées à une artère et les veinules pouvant être reliées à une veine.

On entend par « tissu », un ensemble fonctionnel de cellules et par « organe » un ensemble de tissus remplissant une fonction spécifique.

La méthode de fabrication d'une structure vasculaire comprend au moins :

a) La fabrication d'éléments d'au moins un type, comportant sur leur surface externe des cellules endothéliales et/ou des cellules souches progénitrices endothéliales,
b) L'empilement desdits éléments,
c) L'établissement d'un flux de fluide à travers l'empilement ménageant des canaux le long des lignes de flux.

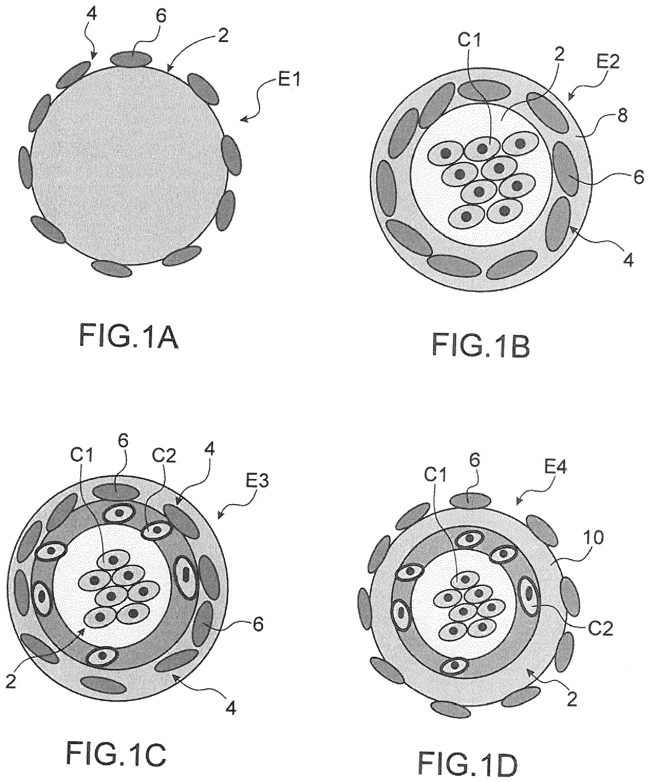

Chacune des étapes va maintenant être décrite en détail.
Un exemple d'éléments E1 fabriqués à l'étape a) est représenté schématiquement sur la figure 1A. Ils comprennent au moins une partie centrale ou noyau 2 en biopolymère et une couche extérieure 4 comprenant des cellules endothéliales et/ou des cellules souches progénitrices endothéliales.

Les cellules endothéliales sont constitutives de l'endothélium qui forme la couche la plus interne des vaisseaux. Les artères et veines comportent trois couches, dont l'endothélium, tandis que les capillaires comportent une seule couche formée par l'endothélium.

Les cellules endothéliales sont des cellules différenciées, i.e. elles sont spécifiques à un organe donné. On parle par exemple de cellules endothéliales lymphatiques et de cellules endothéliales pulmonaires.

Les cellules souches progénitrices endothéliales ne sont pas encore différenciées.

Les cellules endothéliales sont choisies en fonction de l'application, par exemple il s'agit des HMECs (Human Microvascular Endothelial Cells en terminologie anglo-saxonne) ou des cellules endothéliales de foie ou LSECs (Liver sinusoidal endothelial cells en terminologie anglo-saxonne) en co-culture avec des hépatocytes.

A des fins de simplicité, les cellules endothéliales et/ou des cellules souches progénitrices endothéliales seront désignées dans la suite de la description par « cellules endothéliales »

Le noyau 2 comporte un ou plusieurs biopolymères. Un biopolymère est un ensemble de macromolécules biologiques. Les biopolymères utilisables sont bien connus de l'homme du métier et peuvent être des biopolymères naturels ou de synthèse. Ils sont choisis par exemple parmi l'alginate, le collagène, la fibrine, l'élastine, l'acide hyaluronique.

Le ou les biopolymères est ou sont choisis en fonction de la structure vasculaire à fabriquer et en fonction des cellules endothéliales recouvrant le noyau, et du tissu ou de l'organe à fabriquer. Ainsi, en réalisant des éléments comprenant plusieurs couches de biopolymères, on peut former à la fois un milieu favorable aux cellules endothéliales et un ou plusieurs milieux favorables à des cellules du tissu qui auraient plus d'affinité avec un biopolymère différent de celui avec lequel les cellules endothéliales présentent une bonne affinité.

Le ou les biopolymères sont ou non résorbables en fonction de la structure à fabriquer. Le ou les biopolymères sont gélifiés lorsqu'ils forment les éléments. Par exemple on peut prévoir de retransformer le ou les biopolymères en liquide lorsque que la structure vasculaire est terminée afin d'évacuer le ou les biopolymères.

Dans un exemple de réalisation, le noyau comporte uniquement du ou des biopolymères.

Dans un autre exemple le noyau comporte du ou des biopolymères et des cellules typiques de l'organe que l'on souhaite fabriquer.

Les cellules dans le noyau peuvent être de tout type cellulaire, peuvent être des amas de cellules ou organoïdes. Par exemple, les cellules typiques sont des hépatocytes afin de développer un foie-sur-puce, ou des îlots de Langerhans afin de mimer un pancréas-sur-puce.

Les cellules endothéliales recouvrent au moins en partie le noyau 2. Dans cet exemple les cellules endothéliales sont ensemencées sur la surface extérieure du noyau.

En fonction du ou des biopolymères et des cellules endothéliales, il est avantageux de fonctionnaliser le noyau pour faciliter l'accroche des cellules endothéliales sur le noyau. Par exemple dans le cas de l'alginate qui présente peu d'affinité avec les cellules endothéliales, on peut utiliser une protéine améliorant l'ancrage des cellules endothéliales sur le noyau. En variante, le noyau est réalisé avec un mélange de biopolymères dont l'un apporte la tenue mécanique au noyau, tel que l'alginate, et l'autre assure l'affinité avec les cellules endothéliales, tel que le collagène.

Avantageusement le noyau comporte également un facteur de croissance de l'endothélium vasculaire ou VEGF (Vascular endothelial growth factor en terminologie anglo-saxonne) favorisant la prolifération des cellules endothéliales autour du noyau. Les facteurs de croissance peuvent être ajoutés soit directement dans le milieu de perfusion, soit par co-culture de fibroblastes qui vont sécréter des facteurs de croissance. De préférence, les fibroblastes sont placés proches des cellules endothéliales et sont en contact avec elles. Par exemple, les fibroblastes sont sur la couche externe du noyau comme les cellules endothéliales, soit dans une couche entre le noyau et les cellules endothéliales. En variante, les fibroblastes sont placés dans d'autres éléments, libérant les facteurs et permettant la prolifération.

Sur la figure 1B, on peut voir un autre exemple d'éléments E2 dans lequel le noyau comporte des cellules C1, par exemple typiques de l'organe à fabriquer, et la couche extérieure 4 comprend une couche d'encapsulation 8 des cellules endothéliales 6. De préférence la couche d'encapsulation comporte du collagène. En variante, la couche d'encapsulation comporte un mélange de collagène et d'alignate.

Sur la figure 1C, on peut voir un autre exemple d'éléments E3 dans lequel le noyau comporte au cœur des cellules C1 d'un certain type et en périphérie du cœur des cellules C2 d'un autre. Les cellules C1 et C2 sont encapsulées dans les mêmes biopolymères ou des biopolymères différents. Dans cet exemple les cellules endothéliales sont également encapsulées.

L'empilement de couches contenant des types cellulaires différents permet de recréer des tissus stratifiés.

Le nombre de couches contenant des cellules de types différents n'est pas limité à deux. Ainsi il est possible de fournir tous les types cellulaires nécessaires au bon fonctionnement du tissu ou de l'organe que l'on souhaite fabriquer.

Par exemple, les cellules C2 sont des péricytes. En effet les péricytes sont des cellules qui s'étalent au niveau de la paroi externe des capillaires. In vitro, elles aident à rendre le réseau microvasculaire mature. Ainsi en réalisant des éléments comprenant un noyau comportant les cellules tissulaires d'intérêt et des péricytes au niveau de sa couche externe, et des cellules endothéliales sur la surface du noyau, l'architecture des capillaires sanguins est respectée avec le flux circulant au sein d'une paroi composée de cellules endothéliales, avec des péricytes la recouvrant.

Sur la figure 1D, on peut voir un autre exemple d'élément E4 comportant une couche de fonctionnalisation 10 pour faciliter l'accroche des cellules sur le noyau. En variante il peut s'agir d'une couche de biopolymère, typiquement du collagène, choisi en fonction du tissu à réaliser.

Il sera compris que tout type de combinaison entre les biopolymères, les cellules typiques des organes et les cellules endothéliales est envisageable afin de se rapprocher au mieux des tissus ou organes que l'on souhaite reproduire.

Les éléments ont, dans les exemples représentés, des formes sensiblement sphériques. D'autres formes sont envisageables, par exemple la forme ovoïde.

De manière préférée, les dimensions des éléments sont telles que les cellules contenues dans le noyau puissent être perfusées. En effet, la distance de diffusion des nutriments dans le biopolymère est d'environ 200 $\mu$m. Les dimensions des éléments sont donc choisies de sorte que les cellules se trouvent environ au plus à 200 $\mu$m de la surface extérieure du noyau. Par exemple dans le cas d'un élément de forme sphérique, celui-ci a préférentiellement un rayon de l'ordre de 200 $\mu$m. Il sera compris que la distance de diffusion peut varier et dépend du type de biopolymère, de sa densité, du type de nutriments. Par exemple les éléments ont un diamètre inférieur à 500 $\mu$m.

Un exemple de fabrication des éléments E1 va maintenant être décrit.

La fabrication des noyaux peut être réalisée suivant la méthode décrite dans le document Morimoto Y, Onuki M, Takeuchi S. Mass Production of Cell-Laden Calcium Alginate Particles with Centrifugal Force. Adv Healthc Mater. 1 juill 2017;6(13*).

Par exemple on souhaite réaliser un noyau en alginate. L'alginate gélifie par diffusion des ions calcium. On produit des gouttes d'alginate à un diamètre donné, qui tombent ensuite dans un bain de calcium fortement concentré afin de permettre une gélification rapide des gouttes.

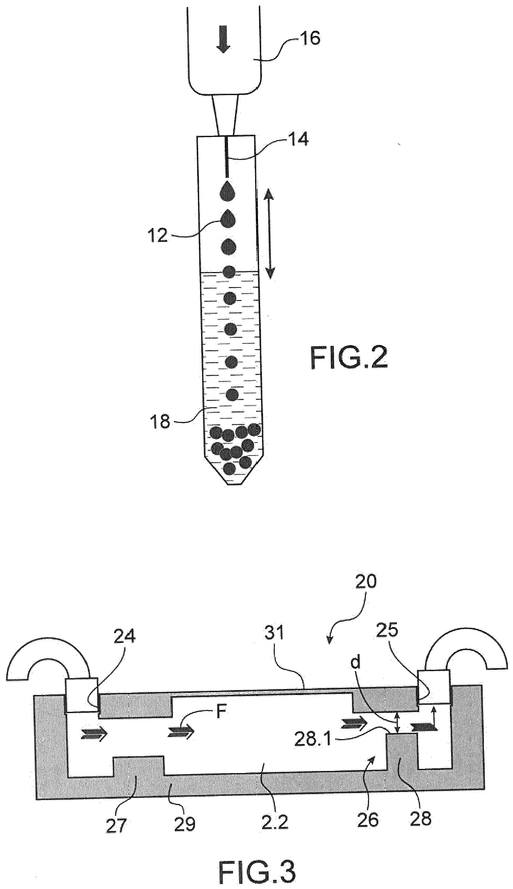

Sur la figure 2, on peut voir une représentation schématique de la réalisation des noyaux en biopolymère.

La formation des gouttes 12 est par exemple obtenue au moyen d'une aiguille 14 montée sur un flacon 16. L'extrémité libre de l'aiguille est située au-dessus du bain de calcium 18 à distance de la surface libre du bain. Le flacon 16 et l'aiguille 14 sont mis en rotation sur eux-mêmes, générant une force centrifuge. Le biopolymère pénètre alors dans l'aiguille et des gouttes de biopolymère se détachent de l'embout de l'aiguille et tombent dans le bain. Lors de leur chute, le biopolymère étant liquide, les gouttes adoptent une forme sphérique. En pénétrant dans le bain de calcium les gouttes gélifient. On obtient alors des microsphères de biopolymère. Les gouttes sont gélifiées et peuvent être utilisées pour être ensemencées avec des cellules endothéliales.

Dans le cas où le noyau comporte des cellules spécifiques aux tissus ou organes à fabriquer, les cellules peuvent être intégrées au noyau ou à une couche externe au noyau par plusieurs techniques. Une technique consiste à mélanger les cellules spécifiques avec le biopolymère constitutif du noyau avant d'être mis dans l'aiguille.

Dans le cas d'éléments multi-couche, une technique consiste à réaliser des premiers éléments, une fois gélifiés ils peuvent être mélangés avec un second biopolymère et des secondes cellules, puis réinjectés dans une aiguille plus grosse pour réaliser une seconde encapsulation. Selon une autre technique, deux flux de polymères en co-écoulement (flow focusing en terminologie anglo-saxonne) sont cisaillés par un flux d'huile. Par exemple on utilise une aiguille contenant le biopolymère du noyau, incluse dans une seconde aiguille plus large contenant le biopolymère de la future couche externe, ce biopolymère contenant ou non des cellules. La centrifugation permet la formation de deux couches en une seule étape, et peut permettre de créer un élément avec une couche externe gélifiée et un cœur liquide.

En variante, des éléments sont réalisés par co-écoulement permettant de réaliser des éléments sectorisés, les différentes zones comportant des cellules identiques ou différentes. Des méthodes de fabrication de réalisation d'éléments par co-écoulement sont par exemple décrites dans le document Agarwal et al. One-step microfluidic generation of pre-hatching embryo-like core-shell microcapsules for miniaturized 3D culture of pluripotent stem cells, LabChip, 2013, 13, 4525-4533 et dans le document Ma et al., Core-shell Hydrogel Microcapsules for Improved Islets Encapsulation, Adv Health Mater. 2013 May; 2(5) pp. 1-12.

Les noyaux et les cellules endothéliales sont ensuite mis en suspension dans un milieu de culture cellulaire afin de permettre aux cellules endothéliales d'adhérer à la surface de noyaux. De préférence, après que les cellules ont adhéré, elles sont laissées en culture afin qu'elles prolifèrent et recouvrent la surface de noyaux de manière sensiblement uniforme. La prolifération peut durer par exemple de 1 jour à quelques jours. En variante, les noyaux et les cellules endothéliales sont directement injectés dans une chambre fluidique qui sera décrite ci-dessous, dans laquelle la prolifération des cellules sur les noyaux et la génération de vaisseaux a lieu.

Il sera compris que le milieu de culture est choisi également pour assurer la survie des cellules à l'intérieur du noyau, et dans le cas de plusieurs types de cellules, le milieu de culture est choisi de sorte à être compatibles avec les différents types de cellule.

Dans le cas d'un biopolymère, tel que l'alginate, présentant peu d'affinité avec les cellules endothéliales, une fonctionnalisation de la surface extérieure des noyaux est avantageusement réalisée pour faciliter l'adhésion des cellules endothéliales. Cette fonctionnalisation est réalisée préalablement à la mise en suspension des noyaux dans le milieu de culture contenant les cellules endothéliales.

Par exemple, cette fonctionnalisation est réalisée en recouvrant les noyaux avec un biopolymère offrant une bonne affinité avec les cellules endothéliales, par exemple le collagène. Pour cela les noyaux sont disposés dans un bain de collagène maintenu froid pour éviter sa gélification. Puis les noyaux sont récupérés et disposés dans un milieu à température permettant au collagène de gélifier et de ne pas endommager les cellules, par exemple entre la température ambiante et 37°C

L'étape b) va maintenant être décrite.

Les éléments réalisés à l'étape a) sont ensuite mis en place dans une chambre fluidique 20 représentée schématiquement sur la figure 3, de sorte à former un empilement poreux.

La chambre fluidique 20 est par exemple formée dans une carte microfluidique et comporte une cavité 22 destinée à recevoir les éléments E1 par exemple, un orifice d'alimentation 24 en fluide situé d'un côté de la cavité, un orifice d'évacuation 25 du fluide situé de l'autre côté de la cavité de sorte que la cavité soit traversée par le flux de fluide. La cavité comporte une extrémité amont située du côté de l'orifice d'entrée et une extrémité aval située du côté de l'orifice de sortie.

L'orifice d'alimentation et l'orifice d'évacuation débouchent avantageusement directement dans l'empilement.

Le fluide assure un apport en nutriments aux cellules et assure également une oxygénation des cellules. La circulation du fluide à travers la cavité est par exemple obtenue au moyen d'une pompe péristaltique. La mise en œuvre d'une pompe péristaltique permet de calibrer précisément le débit du fluide à travers la chambre fluidique et ainsi maîtriser l'effet du flux de fluide sur les cellules endothéliales.

La chambre fluidique comporte, du côté de l'extrémité aval de la cavité, des moyens 26 empêchant les éléments E1 d'être évacués par l'orifice d'évacuation 25.

Dans l'exemple représenté, les moyens 26 comportent une paroi 28 s'étendant transversalement à partir du fond inférieur 29 de la cavité sur une partie de la hauteur de la cavité en amont de l'orifice d'évacuation. La distance entre l'extrémité libre 28.1 de la paroi 28 et le fond supérieur 31 de la cavité est inférieure à la dimension minimale des éléments E1. Dans le cas d'éléments sphériques, la distance d est inférieure au diamètre des éléments, et dans le cas d'éléments ovoïdes, la distance d est inférieure à la plus petite dimension des éléments. En variante, la paroi s'étend du fond supérieur de la chambre, ou d'une paroi latérale de la chambre permettant au fluide de circuler au-dessous et au-dessus de la paroi. La paroi 28 peut être pleine ou perforée. De préférence, la paroi est disposée de sorte à favoriser un flux homogène.

En variante les moyens 26 comporte une grille présentant des trous de dimension inférieure à la dimension minimale des éléments, des piliers distants les uns les autres de sorte à empêcher le passage des éléments en direction de la sortie d'évacuation. Tout autre moyen permettant une rétention des éléments E1 dans la cavité tout en permettant l'établissement d'un flux de fluide entre l'orifice d'alimentation et l'orifice d'évacuation peut être mis en œuvre.

En outre, la cavité est également délimitée du côté de son extrémité aval pour contenir les éléments et assurer le maintien de l'empilement. Dans cet exemple, il s'agit également d'une paroi 27 s'étendant du fond inférieur de la chambre vers le fond supérieur sur une partie de la hauteur de la cavité.

De manière très avantageuse tout ou partie de la chambre est transparent pour permettre de visualiser les éléments au cours du procédé de fabrication.

De manière également très avantageuse, le fond supérieur 31 est amovible permettant de récupérer la structure vasculaire fabriquée. Des moyens d'étanchéité peuvent être prévus entre le fond supérieur et le reste de la chambre fluidique.

Dans l'exemple représenté, le fond supérieur 31 est un film présentant des propriétés adhésives, qui est laminé sur la cavité par exemple réalisée en COC (copolymère cyclo-oléfine), et qui peut ensuite être retiré pour récupérer le tissu ou organe généré.

En variante, le fond supérieur 31 est en matériau rigide, par exemple en COC (copolymère cyclo-oléfine) couplé au reste de la chambre par exemple par aimantation et pouvant ainsi être retiré.

Dans l'exemple représenté, les orifices d'alimentation et d'évacuation sont situés dans le fond supérieur de la chambre. En variante l'un des orifices ou les deux se situe(nt) dans le fond inférieur et/ou dans une ou des parois latérales.

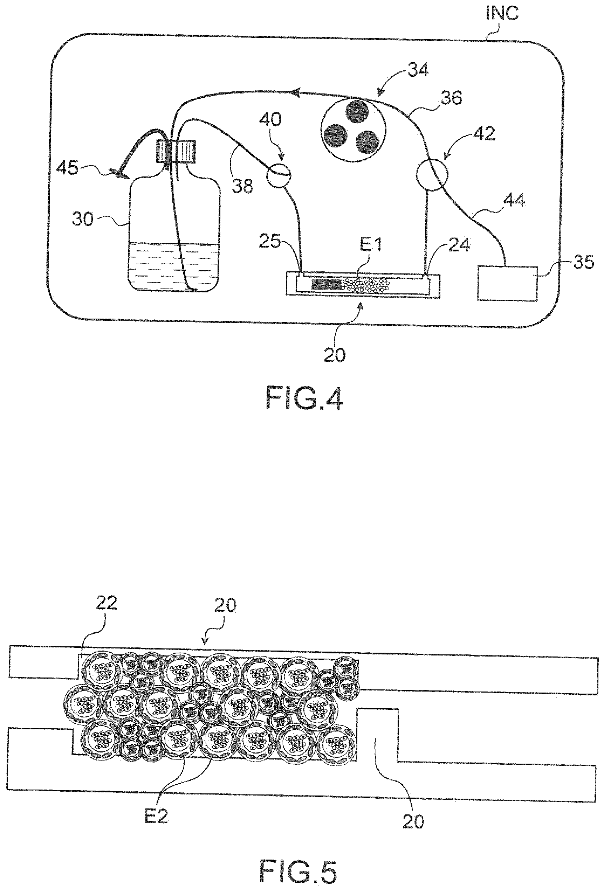

Sur la figure 4, on peut voir une représentation d'un exemple d'un circuit assurant la circulation du fluide dans la chambre fluidique. Dans cet exemple, le circuit est avantageusement un circuit fermé permettant de maintenir un flux continu pendant plusieurs jours.

Le circuit comporte un réservoir de fluide 30, par exemple un flacon, la chambre fluidique, une pompe péristaltique 34 et une poubelle 35. Le circuit comporte également une première connexion fluidique 36 entre le réservoir 30 et l'orifice d'alimentation 24 de la chambre fluidique, sur laquelle est prévue la pompe 34. Une deuxième connexion fluidique 38 est prévue entre l'orifice d'évacuation 25 et le réservoir 30.

De manière avantageuse, une vanne deux voies 40 est prévue sur la deuxième connexion fluidique pour autoriser ou permettre la circulation entre l'orifice de de sortie et le réservoir, et une vanne trois voies 42 est prévue sur la première connexion fluidique 36 permettant la connexion du réservoir 30, soit à l'orifice d'alimentation 24, soit à la poubelle 35 via une troisième connexion fluidique 44. La mise en œuvre des vannes permet d'éviter l'apparition d'un reflux dans la chambre fluidique lorsque la pompe est arrêtée. En effet, en commutant la vanne trois voies 42 de sorte à connecter le réservoir 30 à la poubelle 35 et en commutant la vanne deux voies 40 pour interrompre la connexion entre l'orifice d'évacuation 25 et le réservoir 30, ceci avant d'arrêter la pompe, le reflux est évité.

Les première 36, deuxième 38 et troisième 44 connexions fluidiques sont par exemple réalisées par des tubes microfluidiques

De préférence, le réservoir 30 comporte un filtre 45 permettant l'oxygénation du milieu de culture tout en maintenant le circuit stérile. En variante tout ou partie du circuit et/ou de la carte est poreux à l'oxygène, assurant l'oxygénation du milieu de culture. En variante encore, un mélange gazeux approprié est bullé dans le milieu de culture qui est injecté dans la chambre. Par exemple, la chambre est placée dans un incubateur qui permet la régulation des échanges en établissant par exemple une atmosphère à 5 % de $CO_2$ et environ 80% à 85 % d'humidité.

De préférence le circuit est maintenu à une température de 37°C, par exemple en le plaçant dans un incubateur INC.

Il sera compris que d'autres dispositifs permettant de maîtriser précisément le débit du flux de fluide à travers la chambre fluidique peuvent être mis en œuvre, par exemple en mettant en œuvre un pousse-seringue ou un écoulement par gravité entre deux réservoirs connectés en entrée et sortie dont les niveaux sont différents. Dans le cas du pousse-seringue, l'opérateur doit manipuler les fluides pour maintenir un flux continu. Dans le cas d'un écoulement gravitaire, soit les réservoirs sont suffisamment grands pour ne pas requérir de manipulation de fluide entre le réservoir amont et le réservoir aval, soit l'opérateur transfère du liquide entre les deux réservoirs pour maintenir l'écoulement.

Sur la figure 5, on peut voir la chambre fluidique comportant les éléments E2. Dans cet exemple, l'empilement comporte des éléments de différentes dimensions et éventuellement comportant des cellules ayant des types différents pour faire de la co-culture et/ou de cellules endothéliales différentes. Les éléments sont par exemple injectés ou insérés dans la chambre fluidique de manière à former un empilement relativement compact.

De préférence, les éléments présentent une dimension maximale inférieure à 500 $\mu$m. Suivant l'empilement et les différentes dimensions des éléments, voire les différentes formes des éléments, l'espacement inter-éléments peut être modulé en fonction de l'architecture de la structure vasculaire à fabriquer. L'espacement entre les éléments peut être compris entre quelques $\mu$m et quelques dizaines de $\mu$m, voire une centaine de $\mu$m. Ces valeurs d'espacement correspondent aux diamètres des capillaires, des artérioles et veinules que l'on souhaite reproduire.

L'invention permet d'empiler les éléments en tenant compte de leurs dimensions afin de créer un réseau structuré. De manière avantageuse, on peut prévoir de distribuer les éléments suivant leur taille pour reproduire un réseau vasculaire comportant des artérioles, des veinules reliées par des capillaires. En effet, l'utilisation d'éléments de différentes dimensions permet d'obtenir des empilements différents, afin d'atteindre des pores inter-éléments de dimensions encore plus réduites.

On peut empiler une couche d'éléments de grande taille, puis une couche d'éléments de petite taille, puis à nouveau une couche d'éléments de grosse taille pour former des dimensions de pores d'artérioles, puis de capillaires, puis de veinules.

L'empilement d'éléments de différentes tailles au sein d'une même couche peut permettre d'obtenir des pores inter-éléments de dimensions encore plus réduites.

On peut donc créer des empilements ayant des pores de petites dimensions, afin de se rapprocher de la structure des capillaires sanguins, puis des empilements de pores plus grandes, afin de se rapprocher de la structure des artérioles/veinules de diamètres plus importants

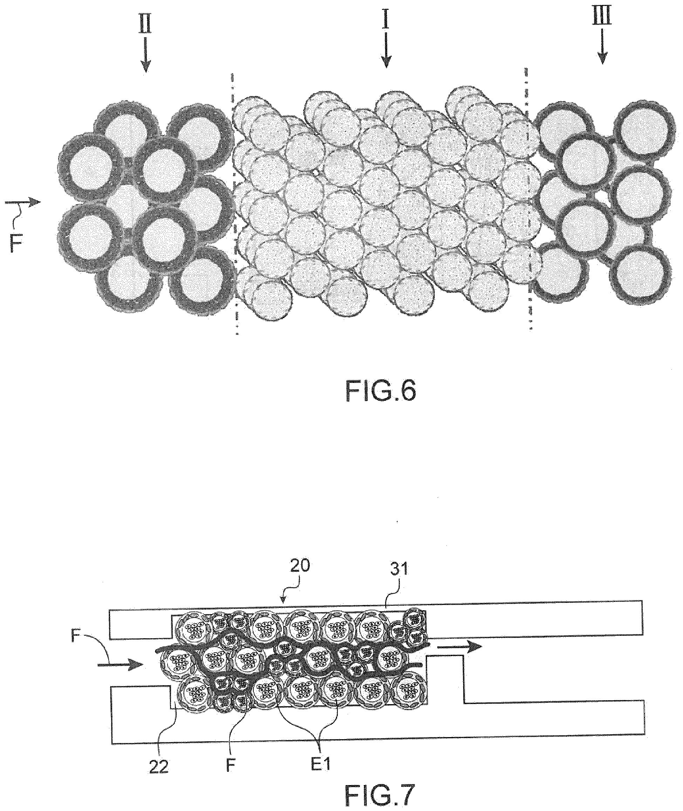

Sur la figure 6, on peut voir un exemple de distribution des éléments permettant la fabrication d'un tel réseau.

Les éléments disposés dans la chambre fluidique en vue de leur perfusion sont répartis en trois empilements I, II et III. Les éléments de l'empilement I sont ceux de plus petit diamètre dont l'empilement offre l'espacement inter-élément le plus faible. Il permet avantageusement de former des capillaires. Les noyaux comportent par exemple des cellules tissulaires et de péricytes et sont recouverts de cellules endothéliales.

L'empilement I est disposé entre les empilements II et III.

Les empilements II et III comportent des éléments de plus grand diamètre et offrent des espacements inter-élément plus grand.

L'empilement II comporte par exemple des éléments comprenant un noyau recouvert d'une couche épaisse de cellules de muscles recouverte de cellules endothéliales, et l'empilement III comporte par exemple des éléments comprenant un noyau recouvert d'une couche fine de cellules de muscles recouverte de cellules endothéliales.

Cette distribution peut être réalisée de sorte à disposer les couches II, I et III les unes au-dessus des autres permettant d'avoir une meilleure tenue lors de l'empilement. De préférence des connexions fluidiques distinctes pour chacun des empilements I, II et III sont prévues pour injecter le fluide dans chacun des empilements séparément. De préférence, une injection a lieu d'abord dans l'empilement II afin que ses éléments forment une première structure vasculaire et se bloquent contre le filtre, puis une injection a lieu dans l'empilement I qui forment une deuxième structure vasculaire qui se bloque contre l'empilement II, puis une injection a lieu dans l'empilement III, formant une troisième structure vasculaire qui se bloque contre la deuxième structure.

En outre en choisissant les dimensions des éléments pour augmenter l'espacement entre les éléments au niveau des extrémités amont et/ou aval de la chambre, et ainsi fabriquer des vaisseaux de plus grand diamètre, il est possible de favoriser l'anastomose.

L'étape c) va maintenant être décrite.

Après mise en place des éléments dans la cavité, un flux de fluide est établi à travers l'empilement entre l'orifice d'entrée et l'orifice de sortie. Les lignes de flux sont schématisées par les flèches F sur la figure 7.

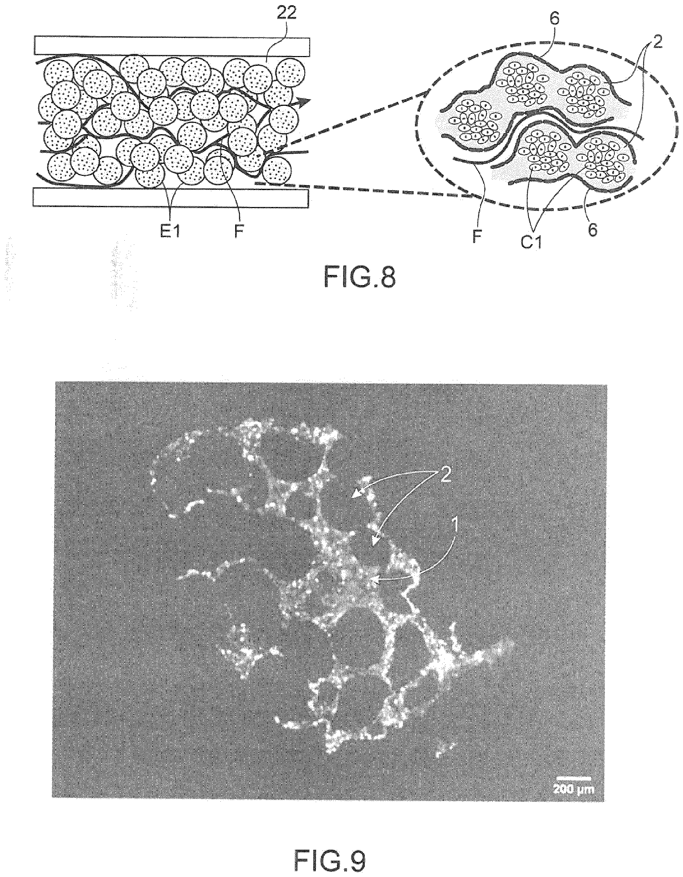

Le flux crée des chemins préférentiels dans l'empilement. Le fait de perfuser l'empilement d'éléments va permettre de créer un réarrangement des cellules endothéliales qui vont s'auto-organiser en tube creux, puis en vaisseaux et capillaires sanguins. Le flux assure un alignement des cellules dans la direction des lignes de flux. Sur la figure 8 on peut voir l'auto-assemblage des cellules endothéliales qui définissent des capillaires.

De manière très avantageuse, le débit de fluide est choisi de sorte à provoquer un cisaillement suffisant à la surface des éléments pour stimuler l'attachement des cellules endothéliales les unes aux autres et favoriser la formation d'endothélium. Par exemple le débit est choisi pour que les contraintes de cisaillement appliquées aux éléments soient comprises entre 1 dyne /cm$^2$ à 10 dynes/cm$^2$, i.e. entre $10^{-5}$ N/cm$^2$ et $10^{-4}$ N/cm$^2$.

Il sera compris que les contraintes des cisaillements varient au sein de l'empilement. La valeur de cisaillement calculée est donc une valeur moyenne au sein de l'empilement.

L'apparition des vaisseaux dans l'empilement d'éléments peut apparaître après plusieurs heures de perfusion par exemple un jour.

On obtient alors des tissus vascularisés qui peuvent être utilisés seuls comme par exemple pour des applications type organe sur puce, dans ce cas les tissus peuvent être conservés dans la chambre fluidique. Alternativement, les tissus vascularisés peuvent être assemblés pour des applications de reconstruction cellulaire, dans ce cas les tissus sont retirés de la chambre.

En fonction de la composition des noyaux et des cellules qu'ils peuvent contenir, les noyaux peuvent soit se résorber, soit rester intègres et participer à la structure vasculaire. Pour supprimer le ou les biopolymères, une étape pour dégélifier le ou les biopolymères peut être prévue, qui à l'état liquide sont évacués par le flux de fluide. Par exemple, à la fin de fabrication de la structure vasculaire, on ajoute dans le fluide une ou plusieurs substances provoquant la dégélification du ou des biopolymères, et leur liquéfaction ; par exemple on peut introduire du citrate de sodium qui dégélifie l'alginate en chelatant le calcium. En maintenant le flux de liquide, le ou les biopolymères sont entraînés par le fluide vers une poubelle. Le fluide peut être injecté dans la structure vasculaire, par exemple dans les capillaires, le citrate passant à travers les parois de capillaires qui sont poreuses, atteint les éléments en biopolymère et provoque leur dissolution. En variante, la chambre comporte dans ses parois des orifices pour pouvoir injecter le citrate dans la cavité à l'extérieur de la structure vasculaire et directement en contact avec les éléments à dissoudre.

On peut prévoir de recouvrir les parois de la cavité avec des cellules endothéliales pour se rapprocher des conditions réelles d'un tissu vascularisé. Ceci permet d'éviter un écoulement du flux majoritairement le long des parois sans cellule. En outre, le flux n'est en contact qu'avec l'endothélium comme c'est le cas dans un système in vivo. De plus, cela permet d'envisager de perfuser avec du sang, l'endothélium évitant la formation de caillots sanguins.

Par exemple, l'entrée et la sortie de la cavité sont formées par des canaux par exemple en matériau plastique ou en verre. De manière avantageuse, ces canaux sont tapissés de cellules endothéliales. Sous perfusion, les cellules endothéliales vont s'assembler et former des vaisseaux dont les diamètres sont ceux des canaux. Ces vaisseaux sont destinés à assurer la connexion de la structure vasculaire à un système vasculaire, par exemple à une artère et à une veine

Dans l'exemple représenté, la cavité est de forme parallélépipédique rectangle. Il en résulte que le tissu vascularisé fabriqué a une forme parallélépipédique rectangle. Afin de se rapprocher de la structure du réseau sanguin au niveau de la subdivision des artères en artérioles puis capillaires, et de leur ramification en veinules, puis veines, on peut avantageusement prévoir d'utiliser une cavité présentant des extrémités amont et aval se rétrécissant en direction de l'orifice d'alimentation et de l'orifice d'évacuation respectivement, par exemple en forme de diamant ou d'ellipse vue de dessus, voire en forme d'organe.

Ces formes présentent l'avantage de faciliter l'anastomose des capillaires avec la paroi des canaux microfluidiques recouverts de cellules endothéliales comme cela a été expliqué ci-dessus.

Comme expliqué ci-dessus, l'application du flux de fluide appliquant un cisaillement de valeur suffisante est favorable à la formation de canaux par les cellules endothéliales. Nous allons maintenant décrire comment les forces de cisaillement appliquées par le flux aux éléments dans la chambre fluidique peuvent être estimées.

Lors du passage d'un fluide au travers d'un canal microfluidique, une force de cisaillement est appliquée au niveau des parois du canal, proportionnelle à la viscosité du liquide, sa vitesse dans la conduite et la section du canal traversé. Pour un milieu poreux, la vitesse du fluide traversant est variable et dépend des propriétés de la structure, comme la porosité, ce qui rend la détermination complexe. En effet, la résolution des équations de continuité de la matière et de Navier-Stoke est très complexe dans de tels milieux de par la complexité de la géométrie. Ainsi, des études ont été réalisées et des modèles ont été établis de manière semi-empirique dans le but de caractériser l'écoulement au sein des milieux poreux.

Les milieux poreux sont définis par un ensemble de particules solides entre lesquelles se trouvent des pores formant l'ensemble des vides. La fraction de vide ε est appelée porosité. Les milieux poreux sont souvent modélisés par des empilements de sphères indéformables contenues dans un canal circulaire.

L'ensemble des paramètres utilisés pour les calculs théoriques sont présentées dans le tableau ci-dessous.

| Paramètres microfluidique | Notation | Unité |
|---|---|---|
| Débit | Q | $[\mu L/s] = [mm^3/s]$ |
| Vitesse en amont de l'empilement | U0 | [mm/s] |
| Vitesse dans l'empilement | U | [mm/s] |
| Section du canal microfluidique | A | $[mm^2]$ |
| Diamètre de la section | D | [mm] |
| Longueur de l'empilement | H | [mm] |
| Porosité | ε | |
| Diamètre des sphères | D | [mm] |
| Diamètre hydraulique | Dh | [mm] |
| Viscosité dynamique du fluide | $\mu$ | $[Po] = [dyn.s/cm^2\}$ |
| Viscosité cinématique du fluide | N | $[m^2/s]$ |

La formule suivante reliant la contrainte de cisaillement moyenne au débit dans la chambre est établie à partir des équations d'Hagen-Poiseuille :

$$\tau = 12 \times \mu \times \frac{(1-\varepsilon)}{\varepsilon} \times \frac{U_o}{d} = 12 \times \mu \times \frac{(1-\varepsilon)}{\varepsilon} \times \frac{Q}{A.d}$$

Lors du procédé de fabrication, le fluide est à 37°C et a une composition proche de l'eau. Nous considérons alors la viscosité dynamique de notre milieu égale à celle de l'eau à 37°C, soit $6,92 \times 10^{-3}$ dyn.s/cm². La section de la chambre utilisée est connue, et le débit de fluide est contrôlé au moyen de la pompe péristaltique. Le diamètre des éléments peut être déterminé au moyen d'un logiciel de traitement d'image tel que ImageJ®. Il est à noter que les éléments réalisés suivant la technique décrite ci-dessus ont une forme d'ellipsoïde, le plus grand diamètre et le plus petit diamètre sont mesurés et une moyenne est réalisée pour obtenir un rayon moyen.

La porosité de l'empilement est à déterminer.

Les théories sur l'empilement de sphères montrent que la porosité minimale pouvant être atteinte par un empilement de sphères rigides est de 26%, obtenue pour une structure hexagonale compacte. Cependant, lorsque le placement des sphères est aléatoire, comme dans le cas de la présente invention, la porosité minimale atteignable est de 36%. Afin de vérifier ce point, un comptage des éléments dans la chambre fluidique est réalisé. Par exemple on utilise une chambre de section 1,8 x 1 mm² remplie d'éléments non recouverts de cellules endothéliales. Le fait d'utiliser d'éléments non recouverts permet l'observation de la totalité de l'empilement car les éléments sont transparents.

Les éléments mesurent 302,5 $\mu$m ± 14,4 $\mu$m. Dans un volume de 1,8 x 2,4 x 1 mm³, il y a 219 éléments, soit une porosité de :

$$\varepsilon = 1 - V\acute{e}l\acute{e}ments/Vchambre = 26,5\%$$

Nous obtenons donc une compacité proche de la structure hexagonale compacte, qui n'est pas atteignable lors de l'empilement aléatoire de sphères indéformables. Ceci s'explique car les éléments sont en réalité déformables, permettant un empilement plus compact.

La contrainte de cisaillement peut alors être calculée avec la formule établie ci-dessus on obtient une contrainte :

$$\tau = 0,50 \text{ dyn/cm}^2$$

pour un débit de 50 µL/min.

Le champ de vitesse et de cisaillement dans l'empilement a été mesuré par micro vélocimétrie par image de particules µPIV (Micro Particle Image Velocimetry en terminologie anglo-saxonne), en appliquant un débit de 50 µl/min. Le cisaillement moyen est de 0,4 à 0,5 dyn/cm$^2$.

Par conséquent la formule du cisaillement établie ci-dessus est vérifiée. Celle-ci peut donc être utilisée pour calculer le débit de fluide à appliquer à travers la chambre pour obtenir un cisaillement moyen donné s'exerçant sur les éléments.

Un exemple de fabrication d'un tissu vasculaire va maintenant être donné.

Lors de l'étape a), on utilise un mélange d'alginate et de collagène, par exemple avec un ratio de 50:50. Le ratio dépend en fait de la concentration initiale des biopolymères. C'est un ratio de 50:50 avec une solution d'alginate initialement à 4% et une de collagène initialement à 6 mg/mL.

Ce mélange présente l'avantage de combiner la rigidité offerte par l'alginate une fois gélifié et l'affinité du collagène avec les cellules endothéliales.

Les cellules endothéliales sont des cellules HMEC-GFP. Ces cellules endothéliales expriment de façon permanente la protéine GFP, ce qui permet leur observation par fluorescence.

Les éléments sont fabriqués selon la méthode décrite ci-dessus.

Le mélange de collagène est mis dans un flacon muni d'une aiguille, auquel est appliquée une force centrifuge. Les gouttes formées tombent dans un bain de calcium qui provoque la gélification de l'alginate. De préférence, le bain d'alginate est froid afin d'éviter la gélification du collagène. Les noyaux sont formés.

Les noyaux sont ensuite disposés dans une solution de collagène de sorte à ce qu'une couche de collagène se forme sur les noyaux afin d'améliorer l'accrochage des cellules endothéliales sur les noyaux. Ensuite la solution est chauffée à 37°C pour provoquer la gélification du collagène.

Dans une étape suivante, les noyaux sont mis en suspension avec les cellules endothéliales dans un milieu de culture cellulaire afin de permettre aux cellules d'adhérer à la surface des noyaux. Une fois que les cellules ont adhéré, les éléments sont laissés en culture afin que les cellules endothéliales prolifèrent et recouvrent la surface des noyaux, par exemple pendant deux jours. Les cellules endothéliales forment alors une couche sensiblement uniforme sur les noyaux.

Les éléments sont prêts à être disposés dans la chambre fluidique.

A l'étape b), les éléments sont injectés dans la chambre fluidique.

A l'étape c), la circulation de fluide à un débit donné est établie à travers la chambre fluidique.

Par exemple le débit est choisi pour appliquer une contrainte de cisaillement moyenne entre 1 dyne/cm$^2$ et 10 dynes/cm$^2$.

Les dimensions des éléments ont été préalablement mesurées par traitement d'image. Puisque les éléments présentent des formes ovoïdes les grands et petits diamètres sont mesurés.

On obtient un diamètre moyen de 338,5 +/-21,0 µm et une sphéricité de 92%.

Les éléments présentent un diamètre inférieur à 400 µm, donc tout point à l'intérieur de l'élément se trouve à moins de 200 µm de la surface. Ainsi, toutes les cellules qui seraient encapsulées dans les éléments auraient accès aux nutriments véhiculés par le futur réseau sanguin.

Les éléments sont par exemple injectés dans la chambre fluidique au moyen d'une pipette. On utilise par exemple le circuit de la figure 4. La pompe péristaltique est réglée pour délivrer le fluide au débit donné pour générer les contraintes de cisaillement souhaitées dans l'empilement. La perfusion se déroule à 37°C par exemple dans une étuve. La pompe est mise en route les vannes deux voies et trois voies étant positionnées de sorte à permettre la circulation du fluide en circuit fermé.

La circulation du fluide est établie.

La porosité déterminée pour des éléments non recouverts de cellules endothéliales est d'environ 0,26 du fait de la déformabilité des éléments. Or les éléments recouverts de cellules endothéliales sont plus rigides, l'empilement obtenu est donc moins dense. On choisit donc comme valeur de porosité ε = 0,36 correspondant à la compacité maximale d'un empilement de sphères indéformables. On choisit alors un débit de 1 ml/min, ce qui génère un cisaillement moyen de 1,21 dyn/cm$^2$.

Les cellules sous l'effet du flux de fluide s'alignent et s'allongent dans la direction de celui-ci. Les cellules endothéliales prolifèrent en établissant des ponts entre les éléments. Après trois jours, la couche de cellules endothéliales devient homogène et des creux entre les cellules ne sont quasiment plus observables.

Sur la figure 9 on peut voir une coupe de la structure obtenue grâce à la méthode selon l'invention après 85 h de perfusion. La structure est découpée en tranches de 10 µm. La photo de la figure 9 est une vue grand champ d'une tranche.

On peut voir les éléments et la couche de cellules endothéliales qui s'est formée entre les éléments.

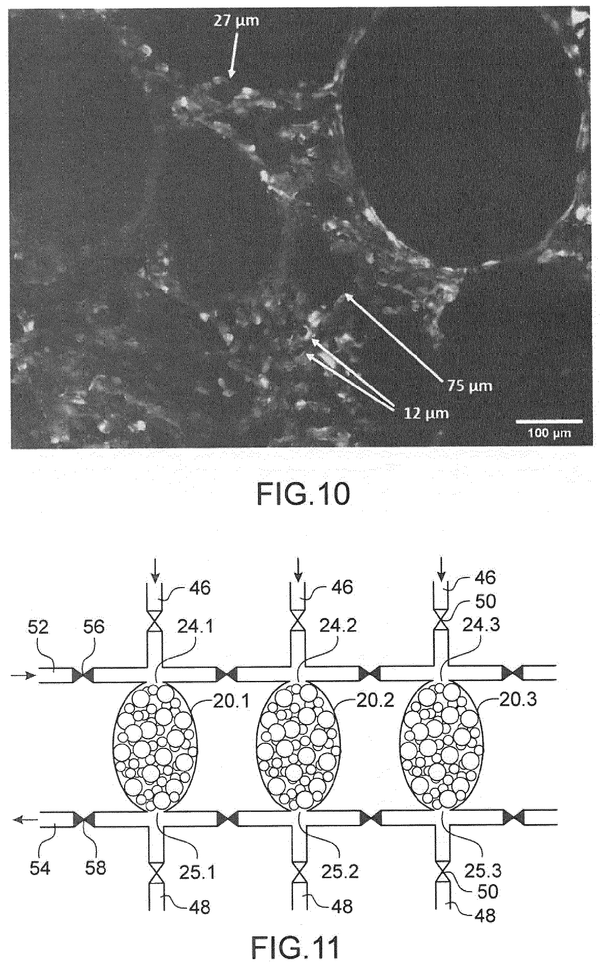

Sur la figure 10, on peut voir une vue agrandie d'une zone inter-éléments. Elle présente des zones vides de taille variable. Par traitement d'images on mesure les diamètres de ces zones. Ils sont compris entre une dizaine de

μm à une centaine de μm. Ces diamètres sont caractéristiques du diamètre physiologique des capillaires sanguins. En outre pendant la perfusion, de la lectine a été ajoutée au fluide, cette lectine a été observée au sein de l'empilement d'éléments, celle-ci s'est fixée aux cellules endothéliales.

Ainsi la méthode de fabrication permet de fabriquer des réseaux de tubes creux, recouverts de cellules endothéliales et perfusable au sein d'un empilement d'éléments de diamètres inférieurs à 400 μm.

Le procédé de fabrication de structures vasculaires présente l'avantage d'appliquer peu de stress aux cellules. En effet celles-ci se trouvent rarement dans un état d'asphyxie. Les cellules, que ce soit les cellules endothéliales ou les cellules typiques de tissu ou d'organe, se trouvent la plupart du temps dans un milieu oxygéné. Ainsi les chances d'obtenir des tissus vascularisés vivants sont très élevées.

En outre le procédé de fabrication offre une grande liberté quant aux dimensions des vaisseaux formés, permettant de se rapprocher au mieux de la structure réelle des tissus. Par ailleurs, contrairement aux techniques de bioimpression, il permet de réaliser des canaux de la taille des capillaires.

La fabrication de tissus vascularisés selon l'invention comporte peu d'étapes et celles-ci sont relativement simples à mettre en œuvre.

Le procédé de fabrication offre en outre la possibilité soit de réaliser un tissu ou organe sur puce qui peut être utilisé pour réaliser des expériences, soit de réaliser des tissus ou organes transplantables sur un être humain par exemple.

Sur la figure 11, on peut voir un exemple de système de fabrication et d'analyse d'un corps sur puce.

Dans cet exemple, plusieurs organes peuvent être fabriqués simultanément en vue de réaliser un corps sur puce, permettant d'étudier l'interaction entre les différents organes d'un corps, par exemple d'un corps humain.

La méthode de fabrication met en œuvre un dispositif comportant des chambres fluidiques 20.1, 20.2, 20.3 comportant des orifices d'alimentation 24.1, 24.2, 24.3 et d'évacuation 25.1, 25.2, 25.3 respectivement, comme dans le cas de la chambre fluidique 20 de la figure 3, permettant l'établissement de flux fluidiques à travers chaque chambre et la fabrication d'organes vascularisés comme cela a été décrit ci-dessus. Les orifices d'alimentation et d'évacuation sont connectés à des canaux 46, 48 reliés à la source de fluide dans le cas d'un circuit fermé. Des vannes 50 sont prévues sur les canaux.

Le dispositif comporte également un canal 52 pour alimenter en parallèle les organes et un canal 54 pour évacuer en parallèle les organes. Des vannes 56, 58 sont prévues sur les canaux 52, 54 entre chaque organe. Le canal 52 remplit la fonction d'une artère, et le canal 54 remplit la fonction d'une veine. Les canaux 52, 54 présentent des diamètres correspondant à ceux des veines et artères.

Ainsi on peut réaliser un système reproduisant au moins en partie les organes et leurs connexions.

Ce système offre l'avantage de ne pas avoir à manipuler les organes entre leur fabrication et leur utilisation en vue de l'analyse médicale. Il suffit de commuter les vannes pour passer de la phase de fabrication à la phase d'exploitation. Les risques de détérioration et/ou pollution sont sensiblement réduits.

Le procédé selon l'invention offre l'avantage de ne pas être limité en taille. En effet il peut être facilement adapté pour réaliser des tissus de grande taille, par exemple des tissus de plusieurs cm$^3$.

La méthode de fabrication peut être utilisée dans le domaine des organes-sur-puces, par exemple pour réaliser des plateformes de test de médicaments, ou de molécules pour des applications cosmétiques, pour la reconstruction de peau par exemple. L'invention permet de réaliser des systèmes adaptables pour tester des nouveaux médicaments, par exemple des molécules de chimiothérapie adaptées au patient par exemple. En outre la fabrication de plusieurs organes sur la même puce permet d'étudier les interactions multi-organes.

La méthode de fabrication peut également être utilisée pour réaliser des greffes de tissus. La méthode permet de réaliser une greffe dont la revascularisation avec le système du patient serait plus efficace et donc améliorée.

La méthode de fabrication permet également le développement d'organes. Par exemple, la méthode peut permettre de générer un pancréas vascularisé, utilisant des éléments contenant des îlots pancréatiques qui libèrent l'insuline en fonction du taux de glucose dans le sang, et dans laquelle le réseau vasculaire en périphérie des éléments serait relié au réseau vasculaire du patient. De même, la méthode selon l'invention peut permettre de réaliser un foie avec des éléments contenant des hépatocytes. La greffe de foie en serait facilitée.

## Revendications

1.  Méthode de fabrication d'une structure vasculaire comportant :

    a) la fabrication d'éléments comprenant au moins un noyau comprenant au moins un biopolymère, et des cellules endothéliales et/ou des cellules souches progénétrices endothéliales sur sa périphérie externe,
    b) mise en place desdits éléments dans une chambre fluidique de sorte à ce qu'ils forment au moins un empilement,

c) l'établissement d'un flux de fluide assurant un apport de nutriments et une oxygénation à travers l'empilement de sorte à ménager des canaux entre les éléments le long des lignes de flux, le flux de fluide présentant un débit tel que les éléments subissent une contrainte de cisaillement moyenne comprise entre 0,1 dyne/cm$^2$ et 100 dynes/cm$^2$, préférentiellement entre 1 dyne/cm$^2$ et 10 dynes/cm$^2$.

2. Méthode de fabrication selon la revendication 1, dans laquelle lors de l'étape a) des cellules, à l'exclusion de cellules souches embryonnaires humaines, sont introduites dans le noyau.

3. Méthode de fabrication selon la revendication 2, dans laquelle le noyau comporte au moins un cœur contenant au moins une partie desdites cellules et une couche externe contenant une autre partie desdites cellules, les cellules du cœur et de la couche externe étant du même type ou non, la couche externe contenant avantageusement des péricytes.

4. Méthode de fabrication selon l'une des revendications 1 à 3, dans laquelle les éléments ont une plus grande dimension inférieure à 1 mm, avantageusement inférieure à 500 μm.

5. Méthode de fabrication selon l'une des revendications 1 à 4, dans laquelle les éléments sont disposés dans un milieu de culture avant l'étape b) pour permettre la prolifération des cellules endothéliales et/ou des cellules souches progénétrices endothéliales et le recouvrement total ou partiel des noyaux.

6. Méthode de fabrication selon l'une des revendications 1 à 5, dans laquelle lors de l'étape b) des éléments des dimensions et/ou de forme différentes sont mis en place.

7. Méthode de fabrication selon l'une des revendications 1 à 6, dans laquelle lors de l'étape b), des éléments de dimensions différentes sont répartis en plusieurs empilements successifs dans la direction du flux.

8. Méthode de fabrication selon la revendication 7, dans laquelle un empilement de premiers éléments présentant un espacement inter-éléments de l'ordre du diamètre des capillaires est disposé entre deux empilements de deuxièmes éléments présentant un espacement inter-élément de l'ordre du diamètre des artérioles ou veinules.

9. Méthode de fabrication selon l'une des revendications 1 à 8, dans laquelle, préalablement à la mise en places des d'éléments dans la chambre fluidique, des cellules endothéliales et/ou des cellules souches progénétrices endothéliales recouvrent les parois de la chambre et/ou des canaux d'entrée et/ou sortie de fluide.

10. Système de fabrication d'une structure vasculaire pour la mise en œuvre de la méthode de fabrication selon l'une des revendications précédentes comportant n chambres fluidiques (20), n ≥ 1, munies d'un orifice d'alimentation (24) en fluide et d'un orifice d'évacuation en fluide, une cavité disposée entre l'orifice d'alimentation et l'orifice d'évacuation (25) et configurée pour recevoir les éléments, et des moyens (26) pour maintenir les éléments dans la cavité, une réservoir (30) de fluide connecté au moins à l'entrée d'alimentation et des moyens de circulation pour assurer la circulation du fluide à travers la cavité, les moyens de circulation du fluide étant tels qu'ils établissent un flux de fluide présentant un débit tel que les éléments subissent une contrainte de cisaillement moyenne comprise entre 0,1 dyne/cm$^2$ et 100 dynes/cm$^2$, préférentiellement entre 1 dyne/cm$^2$ et 10 dynes/cm$^2$.

11. Système de fabrication selon la revendication précédente, dans lequel le réservoir (30) est connecté à l'orifice d'évacuation (25) de sorte à former un circuit fermé.

12. Système de fabrication selon la revendication précédente, comportant des moyens (40, 42) pour isoler la chambre fluidique avant l'arrêt des moyens de circulation et/ou des moyens pour oxygéner le fluide.

13. Système de fabrication selon l'une des revendications 10 à 12, dans lequel chaque chambre fluidique comporte une partie amovible pour permettre d'accéder à la cavité et retirer la structure vasculaire.

14. Système de fabrication selon l'une des revendications 10 à 13, dans lequel la cavité comporte une partie centrale et des extrémités amont et aval en considérant le sens d'écoulement du fluide, les extrémités amont et aval présentant une section transversale se réduisant en direction des orifices d'alimentation et d'évacuation.

15. Système de fabrication selon l'une des revendications 10 à 14, dans lequel n est supérieur ou égal à 2, et dans lequel le système de fabrication comporte un premier canal connectant en parallèle les chambres fluidiques et un deuxième canal connectant en parallèle les chambres fluidiques, le premier canal et le deuxième canal ayant des diamètres de l'ordre de ceux des artères et des veines.

FIG.1A

FIG.1B

FIG.1C

FIG.1D

FIG.2

FIG.3

FIG.4

FIG.5

FIG.6

FIG.7

FIG.8

FIG.9

FIG.10

FIG.11

Europäisches
Patentamt

European
Patent Office

Office européen
des brevets

**RAPPORT DE RECHERCHE EUROPEENNE**

Numéro de la demande

EP 20 20 0325

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 03/078586 A2 (UNIV CALIFORNIA [US]; GEORGE STEVEN C [US] ET AL.) 25 septembre 2003 (2003-09-25) * revendications 10,11; figures 9A, 9B; exemple 5 * | 1-15 | INV. C12M3/00 C12M3/06 C12M1/12 C12M1/00 C12M1/42 C12N5/00 |
| A | WO 2019/148013 A2 (TEXAS A & M UNIV SYS [US]) 1 août 2019 (2019-08-01) * alinéa [0029]; figure 1 * | 1-15 | |
| A | WO 2018/020274 A1 (UNIV CENTRAL LANCASHIRE [GB]) 1 février 2018 (2018-02-01) * figure 2b; tableau 1 * | 1-15 | |
| A | EP 3 514 228 A1 (REVOTEK CO LTD [CN]) 24 juillet 2019 (2019-07-24) * revendications 1-4 * | 1-15 | |

DOMAINES TECHNIQUES
RECHERCHES (IPC)

C12M
C12N

Le présent rapport a été établi pour toutes les revendications

| Lieu de la recherche | Date d'achèvement de la recherche | Examinateur |
|---|---|---|
| Berlin | 15 février 2021 | Jones, Laura |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
............................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 20 20 0325

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-02-2021

| Document brevet cité au rapport de recherche | | Date de publication | Membre(s) de la famille de brevet(s) | | Date de publication |
|---|---|---|---|---|---|
| WO 03078586 | A2 | 25-09-2003 | AU | 2003214155 A1 | 29-09-2003 |
| | | | US | 2006018838 A1 | 26-01-2006 |
| | | | WO | 03078586 A2 | 25-09-2003 |
| WO 2019148013 | A2 | 01-08-2019 | US | 2020360929 A1 | 19-11-2020 |
| | | | WO | 2019148013 A2 | 01-08-2019 |
| WO 2018020274 | A1 | 01-02-2018 | CN | 109790520 A | 21-05-2019 |
| | | | EP | 3491125 A1 | 05-06-2019 |
| | | | US | 2020339956 A1 | 29-10-2020 |
| | | | WO | 2018020274 A1 | 01-02-2018 |
| EP 3514228 | A1 | 24-07-2019 | CN | 108138134 A | 08-06-2018 |
| | | | CN | 109735434 A | 10-05-2019 |
| | | | CN | 109880795 A | 14-06-2019 |
| | | | CN | 109913400 A | 21-06-2019 |
| | | | CN | 109913401 A | 21-06-2019 |
| | | | CN | 109913402 A | 21-06-2019 |
| | | | CN | 109943518 A | 28-06-2019 |
| | | | CN | 109943519 A | 28-06-2019 |
| | | | CN | 110093304 A | 06-08-2019 |
| | | | CN | 110129253 A | 16-08-2019 |
| | | | CN | 110205279 A | 06-09-2019 |
| | | | CN | 110205280 A | 06-09-2019 |
| | | | CN | 110205281 A | 06-09-2019 |
| | | | CN | 110229778 A | 13-09-2019 |
| | | | CN | 110229779 A | 13-09-2019 |
| | | | CN | 110331123 A | 15-10-2019 |
| | | | EP | 3514228 A1 | 24-07-2019 |
| | | | JP | 6755952 B2 | 16-09-2020 |
| | | | JP | 2019531695 A | 07-11-2019 |
| | | | JP | 2020202856 A | 24-12-2020 |
| | | | US | 2019328935 A1 | 31-10-2019 |
| | | | WO | 2018050092 A1 | 22-03-2018 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MORIMOTO Y ; ONUKI M ; TAKEUCHI S.** Mass Production of Cell-Laden Calcium Alginate Particles with Centrifugal Force. *Adv Healthc Mater.,* 01 Juillet 2017, vol. 6 (13

- **AGARWAL et al.** One-step microfluidic generation of pre-hatching embryo-like core-shell microcapsules for miniaturized 3D culture of pluripotent stem cells. *LabChip,* 2013, vol. 13, 4525-4533
- **MA et al.** Core-shell Hydrogel Microcapsules for Improved Islets Encapsulation. *Adv Health Mater.,* Mai 2013, vol. 2 (5), 1-12